# EUROPÄISCHE PATENTSCHRIFT

(11) **EP 1 795 156 B1**
(45) Veröffentlichungstag und Bekanntmachung des Hinweises auf die Patenterteilung: **13.02.2008**
(21) Anmeldenummer: 06023893.8
(22) Anmeldetag: 17.11.2006
(51) Int. Cl.: A61F 5/01

(54) **Unterschenkelorthese**
Lower leg orthosis
Orthèse de jambe

(30) Priorität: 08.12.2005 DE 102005058999
(43) Veröffentlichungstag der Anmeldung: 13.06.2007
(73) Patentinhaber: Otto Bock HealthCare IP GmbH & Co. KG, 37115 Duderstadt (DE)
(72) Erfinder: Körner, Doris, 37085 Göttingen (DE); von Ascheberg, Alexander, 37115 Duderstadt (DE)
(74) Vertreter: Lins, Edgar

(56) Entgegenhaltungen:
- WO-A-01/01896
- WO-A-02/096328
- GB-A- 2 139 089
- US-B1- 6 302 858
- US-B1- 6 406 500

## Beschreibung

Die Erfindung betrifft eine Unterschenkelorthese mit Befestigungseinrichtungen für eine Befestigung der Orthese am Fuß und am Unterschenkel und mit einer L-förmigen, aus einem federnden Material gebildeten Stützfeder, die einen aufrechten, eine Anlageebene definierenden, zum Anliegen an der Rückseite des Unterschenkels vorgesehenen Schenkel aufweist, an dessen unteres Ende sich ein zum Untergreifen des Fußes ausgebildeter horizontaler Schenkel anschließt.

Eine derartige Bauform einer Unterschenkelorthese ist seit langem bekannt, siehe zum Beispiele Dokument GB-A-2 139 089. Das die Stützfeder bildende federnde Flachmaterial kann dabei durch einen Federstahl, vorzugsweise aber durch einen faserverstärkten Kunststoff gebildet sein.

Die aus einem federnden Flachmaterial bestehenden Stützfedern bilden in seitlicher Ansicht einen im Wesentlichen rechten Winkel, wobei sowohl der aufrechte Schenkel als auch der horizontale Schenkel Biegungen aus der Ebene des Flachmaterials heraus aufweisen, um Anpassungen an die anatomischen Gegebenheiten vorzunehmen. Darüber hinaus ist der Übergang zwischen dem aufrechten Schenkel und dem horizontalen Schenkel abgerundet. Die Stützfeder ist bezüglich ihrer Schenkel symmetrisch zu einer aufrechten Mittenebene ausgebildet, was sich aufgrund der Verwendung eines federnden Flachmaterials ohne weiteres ergibt.

Die Anatomie des Fußgelenks führt dazu, dass sich im ruhenden Stand eine Außenstellung des Fußes ausbildet, die größenordnungsmäßig 7° gegenüber der Sagittalebene beträgt. Im Bewegungsablauf des Schrittzyklus entsteht eine Rotation des Unterschenkels (Tibiarotation), die die Außenstellung des Fußes noch verstärkt, sodass diese 7 bis 12° oder mehr betragen kann. Dieser Außenstellung des Fußes wird durch bekannte, einfach aufgebaute und funktionsfähige Orthesen nicht Rechnung getragen.

Der Erfindung liegt daher die Aufgabe zugrunde, eine einfach aufgebaute Unterschenkelorthese der eingangs erwähnten Art so auszubilden, dass bei Erhalt einer guten Funktionsfähigkeit der natürlichen Außenstellung des Fußes Rechnung getragen wird.

Diese Aufgabe wird erfindungsgemäß bei einer Unterschenkelorthese der eingangs erwähnten Art dadurch gelöst, dass der aufrechte Schenkel im untern Bereich in sich derart verwunden ist, dass der das untere Ende fortsetzende horizontale Schenkel aus der senkrecht zur Anlageebene des aufrechten Schenkels oberhalb des in sich verwundenden unteren Bereichs stehende Ebene um einen Winkel α herausgedreht ist.

Die durch den aufrechten Schenkel definierte Anlageebene liegt regelmäßig parallel zur Frontalebene des Patienten. Erfindungsgemäß wird der aufrechte Schenkel über einen unteren Bereich, der den Knöchelbereich einschließt allmählich so tordiert, dass sich der senkrecht zum unteren Ende anschließende horizontale Schenkel in der gewünschten Außenstellung befindet. Die sich etwa am unteren Ende des aufrechten Schenkels befindliche gedachte Biegelinie, um die sich der horizontale Schenkel relativ zum aufrechten Schenkel bei Belastung aufgrund der Elastizität des Materials bewegen kann, liegt somit nicht in der Anlageebene (Frontalebene) sondern ist bereits um den Winkel α aus dieser Ebene herausgedreht. Dadurch ist sichergestellt, dass sich der horizontale Schenkel weiterhin senkrecht zum aufrechten Schenkel am unteren Ende anschließen kann.

Dies ist insbesondere wichtig, wenn ein faserverstärkter Kunststoff verwendet wird, dessen Faserverbund in Längsrichtung der Stützfeder parallel zueinander verlaufende Fasern aufweist, die bei einer Abwinkelung aus einer Biegeebene heraus (durch die Bewegung des horizontalen Schenkels relativ zum aufrechten Schenkel) ansonsten beim Abrollen gegeneinander bewegt werden würden und eine Delamination des faserverstärkten Kunststoffmaterials bewirken könnten.

Erfindungsgemäß ist somit der aufrechte Schenkel, der im oberen Bereich eine senkrecht zu einer Mittenebene (Sagittalebene) liegende Anlageebene definiert, im unteren Bereich etwa so tordiert, dass der senkrecht zu dem aufrechten Schenkel am unteren Ende stehende horizontale Schenkel den Winkel zur mittigen Unterstützung des Fußes in der natürlichen Außenstellung einnimmt. Der aufrechte Schenkel kann somit mittig auf der Rückseite des Unterschenkels angeordnet werden. In einem unteren Bereich (Biegebereich), in dem der aufrechte Schenkel auf der Höhe der Achillessehne verläuft, findet dann die Verwindung statt, durch die die Außenstellung des horizontalen Schenkels bewirkt wird. Die Fußaußenstellung wird somit an der Stützfeder im Bereich des aufrechten Schenkels berücksichtigt, sodass die Biegelinie zwischen horizontalem Schenkel und aufrechtem Schenkel bereits aus der Anlageebene um den Winkel der gewünschten Fußaußenstellung herausgedreht ist.

Das Material der Stützfeder ist in einer bevorzugten einfachen Ausführungsform ein Flachmaterial, dessen große Oberseite im oberen Bereich des aufrechten Schenkels die Anlageebene definiert und im unteren Bereich um den Winkel α gegenüber der Anlagebene verwunden ist, sodass sich der horizontale Schenkel senkrecht zu der Oberfläche des aufrechten Schenkels am unteren Ende anschließen kann und dabei den gewünschten Winkel für die Fußaußenstellung einnimmt.

Obwohl sich somit die erfindungsgemäße Orthese um eine gedachte Biegelinie zwischen aufrechtem Schenkel und horizontalem Schenkel beim Abrollen verformt, wodurch das Material der Orthese geschont wird, resultiert eine Bewegung zwischen Unterschenkel und Fuß, die sich über die Anlageebene des aufrechten Schenkels überträgt, sodass die Bewegungsebene für den gestützten Fuß parallel zur Frontalebene verläuft und damit der natürlichen Bewegung im Knöchelgelenk entspricht.

Die Erfindung soll im Folgenden anhand eines in der Zeichnung dargestellten Ausführungsbeispiels näher erläutert werden. Es zeigen:
- Figur 1: eine schematische Darstellung einer erfindungsgemäßen Stützfeder in Relation zu einem Fuß des Orthesenträgers;
- Figur 2: eine Seitenansicht der Stützfeder;
- Figur 3: eine Frontalansicht der Stützfeder;
- Figur 4: eine schematische Erläuterung der bewirkten Fußaußenstellung;
- Figur 5: eine schematische Darstellung einer mit einer erfindungsgemäßen Stützfeder aufgebauten Unterschenkelorthese.

In der Zeichnung sind die eine Stützfeder 1 zu einer Unterschenkelorthese ergänzenden Befestigungsmittel für die Befestigung am Unterschenkel und am Fuß aus Gründen der Übersichtlichkeit nicht dargestellt. Hierfür sind zahlreiche Ausführungsformen dem Fachmann bekannt. Wie die Zeichnung verdeutlicht, besteht die Stützfeder 1 aus einem aufrechten Schenkel 2 und einem im Wesentlichen horizontalen Schenkel 3, der zum Untergreifen eines Fußes 4 des Orthesenträgers bestimmt ist.

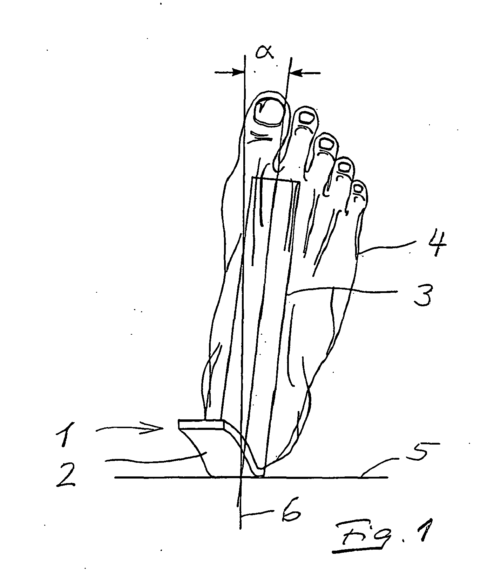

In Figur 1 ist eine Frontalebene 5 und eine Sagittalebene 6 des Orthesenträgers angedeutet. Der aufrechte Schenkel 2 liegt mit seinem oberen Ende parallel zur Frontalebene 5, zu der die Sagittalebene 6 senkrecht steht. Der Fuß 4 des Orthesenträgers nimmt im beidbeinigen Stand die in Figur 1 dargestellte Position ein, ist somit mit seiner Längsachse um einen Winkel α, der beispielsweise 7° beträgt, nach außen gedreht.

Dementsprechend ist der horizontale Schenkel 3 der Stützfeder 1 ebenfalls um den Winkel α nach außen gedreht.

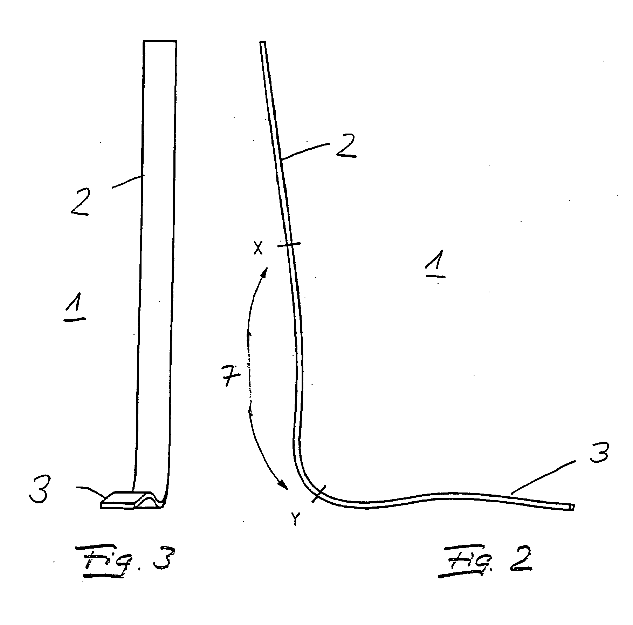

Wie die Figuren 2 und 3 verdeutlichen, geschieht die Verdrehung des horizontalen Schenkels 3 dadurch, dass der aufrechte Schenkel 2 in einem unteren Bereich 7, der zwischen den in Figur 2 eingezeichneten Punkten X und Y liegt, in sich etwas um die Hochachse schraubenförmig gedreht ist. Der aufrechte Schenkel 2 geht ab dem Punkt X (Beginn des Biegebereichs) in eine Torsion über, durch die die Drehung um den Winkel α bis zum Punkt Y realisiert wird. Am Punkt Y geht der aufrechte Schenkel 2 in den horizontalen Schenkel 3 ohne eine Abwinkelung über.

Die Zeichnung verdeutlicht ferner, dass in dem Biegebereich 7 der aufrechte Schenkel eine von vorn gesehene konkave Wölbung aufweist, die dem Verlauf im Fesselbereich eines Fußes nachempfunden ist. Demgegenüber ist der Übergangsbereich (um den Übergangspunkt Y herum) konvex gewölbt, um einen gleichmäßigen Übergang für die longitudinal verlaufenden Fasern im federnden Flachmaterial zu gewährleisten. Der horizontale Schenkel 3 weist - von oben gesehen - eine konvexe Wölbung auf, die dem Verlauf der Fußsohle des Fußes 4 entspricht.

In dem dargestellten Ausführungsbeispiel ist das Material der Stützfeder 1 ein Flachmaterial, das vorzugsweise eine Breite von 15 bis 40 mm und eine Stärke von 2 bis 12 mm aufweist. Der Biegebereich 7 sollte eine gewisse Mindesthöhe nicht unterschreiten, damit die Verwindung in sich gleichmäßig und allmählich erfolgt und nicht zu einer Schwächung der Statik des aufrechten Schenkels 2 führt. Die Länge des Biegebereichs liegt vorzugsweise zwischen 3 und 20 cm.

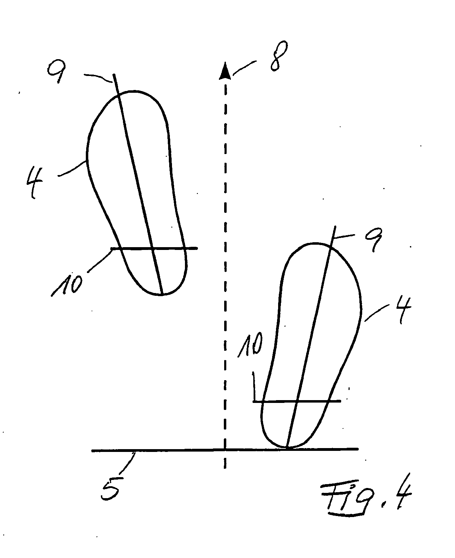

Oberhalb des Biegebereichs 7 definiert die Orientierung des Materials der Stützfeder 1 die Anlageebene für den Unterschenkel. Diese Anlageebene liegt im Wesentlichen parallel zur Frontalebene des Patienten, wie dies in Figur 4 verdeutlicht ist.

Figur 4 zeigt schematisch eine Laufrichtung 8 eines Menschen beim Gehen. Die Laufrichtung wird im Wesentlichen durch die Sagittalebene des menschlichen Körpers bestimmt. Die Füße 4 bewegen sich beiderseits der durch die Laufrichtung 8 bestimmten Ebene, wobei beim Auftreten die Fußaußenstellung realisiert wird, sodass sich eine zur Laufrichtung 8 schräge Fußachse 9 ergbit.

Entsprechend der Lage der natürlichen Knöchelachse wird durch die Orthese eine Drehachse 10 für das Abrollen des Fußes 4 unterstützt, die parallel zur Frontalebene 5 liegt. Die Drehachse 10 ergibt sich aus der Bewegung der oberhalb des Biegebereichs 7 ausgebildeten Anlageebene des aufrechten Schenkels 2 in seiner Bewegung relativ zu dem horizontalen Schenkel 3.

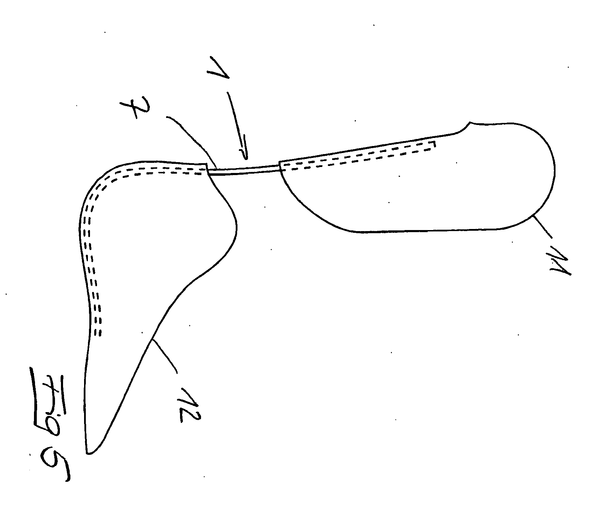

Die in Figur 5 dargestellte, mit der erfindungsgemäßen Stützfeder 1 aufgebaute Orthese wird durch eine Befestigungsschale 11 für den Wadenbereich des Unterschenkels und eine Befestigungsschale 12 für den Fußbereich ergänzt. Mit den Befestigungsschalen 11, 12 ist die Stützfeder 1 drehfest verbunden. Weitere Befestigungsmittel, beispielsweise in Form von Klettbändern zur Fixierung des Unterschenkels bzw. des Fußes an den Befestigungsschalen 11, 12 sind nicht dargestellt.

In dem Ausführungsbeispiel gemäß Figur 5 erstreckt sich die Befestigungsschale 11 für den Unterschenkel etwa bis zum Beginn des Biegebereichs 7 der Stützfeder 1, deckt somit im Wesentlichen die Anlageebene für den Unterschenkel ab.

## Patentansprüche

1. Unterschenkelorthese mit Befestigungseinrichtungen für eine Befestigung der Orthese am Fuß und am Unterschenkel und mit einer L-förmigen, aus einem federnden Material gebildeten Stützfeder (1), die einen aufrechten, eine Anlageebene (5) definierenden, zum Anliegen an der Rückseite des Unterschenkels vorgesehenen Schenkel (2) aufweist, an dessen unteres Ende sich ein zum Untergreifen des Fußes (4) ausgebildeter horizontaler Schenkel (3) anschließt, **dadurch gekennzeichnet, dass** der aufrechte Schenkel (2) im untern Bereich (7) in sich derart verwunden ist, dass der das untere Ende fortsetzende horizontale Schenkel (3) aus der senkrecht zur Anlageebene (5) des aufrechten Schenkels oberhalb des in sich verwundenden unteren Bereichs (7) stehende Ebene (6) um einen Winkel α herausgedreht ist.

2. Unterschenkelorthese nach Anspruch 1, **dadurch gekennzeichnet, dass** das Material der Stützfeder (1) ein faserverstärkter Kunststoff ist.

3. Stützfeder nach Anspruch 2, **dadurch gekennzeichnet, dass** der faserverstärkte Kunststoff einen Faserverbund bildet, der sich über die Länge der Stützfeder (1) erstreckende, zueinander parallel verlaufende Fasern aufweist.

4. Unterschenkelorthese nach Anspruch 2 oder 3, **dadurch gekennzeichnet, dass** die Fasern Carbonfasern sind.

5. Unterschenkelorthese nach Anspruch 2 oder 3, **dadurch gekennzeichnet, dass** die Fasern Glasfasern sind.

6. Unterschenkelorthese nach Anspruch 2 oder 3, **dadurch gekennzeichnet, dass** die Fasern Polyesterfasern sind.

7. Unterschenkelorthese nach einen der Ansprüche 2 bis 6, **dadurch gekennzeichnet, dass** die Fasern aus einer Mischung unterschiedlicher Faserarten bestehen.

8. Unterschenkelorthese nach einem der Ansprüche 1 bis 7, **dadurch gekennzeichnet, dass** das Material der Stützfeder (1) ein Flachmaterial ist.

## Claims

1. A below-knee orthosis with fastening devices for fastening the orthosis to the foot and to the lower leg, and with an L-shaped support spring (1) which is made of a resilient material and which has an upright branch (2) that defines a contact plane (5) and is intended to bear on the dorsal aspect of the lower leg, the lower end of which upright branch is adjoined by a horizontal branch (3) designed to engage under the foot (4), wherein the upright branch (2) is twisted in the lower area (7) in such a way that the horizontal branch (3) continuing the lower end is turned outward by an angle α from the plane (6) lying perpendicular to the contact plane (5) of the upright branch above the twisted lower area (7).

2. The below-knee orthosis as claimed in claim 1, wherein the material of the support spring (1) is a fiber-reinforced plastic.

3. The support spring as claimed in claim 2, wherein the fiber-reinforced plastic forms a fiber composite that comprises mutually parallel fibers extending along the length of the support spring (1) .

4. The below-knee orthosis as claimed in claim 2 or 3, wherein the fibers are carbon fibers.

5. The below-knee orthosis as claimed in claim 2 or 3, wherein the fibers are glass fibers.

6. The below-knee orthosis as claimed in claim 2 or 3, wherein the fibers are polyester fibers.

7. The below-knee orthosis as claimed in one of claims 2 through 6, wherein the fibers are composed of a mixture of different fiber types.

8. The below-knee orthosis as claimed in one of claims 1 through 7, wherein the material of the support spring (1) is a flat material.

## Revendications

1. - Orthèse de jambe comprenant des dispositifs de fixation pour fixer l'orthèse au pied et à la jambe et comportant un ressort d'appui (1) en forme de L réalisé en matière élastique, présentant une branche (2) verticale, définissant un plan d'appui (5) destinée à s'appuyer contre le côté dorsal de la jambe, dont l'extrémité inférieure se raccorde à une branche (3) réalisée horizontalement pour s'engager sous le pied (4), **caractérisée en ce que** la branche verticale (2) est dans sa zone inférieure (7) tordue de telle sorte que la branche horizontale (3) qui prolonge la partie inférieure de la branche verticale est tournée vers l'extérieur suivant un angle α par rapport à un plan (6) perpendiculaire au plan d'appui (5) situé au dessus de la zone inférieure tordue.

2. - Orthèse de jambe selon la revendication 1, **caractérisée en ce que** le matériau du ressort d'appui (1) est une matière synthétique renforcée de fibres.

3. **-** Orthèse de jambe selon la revendication 2, **caractérisée en ce que** la matière synthétique renforcée de fibres est un composite de fibres qui présente des fibres parallèles les unes aux autres s'étendant suivant la longueur du ressort d'appui (1).

4. - Orthèse de jambe selon la revendication 2 ou 3, **caractérisée en ce que** les fibres sont des fibres de carbone.

5. - Orthèse de jambe selon la revendication 2 ou 3, **caractérisée en ce que** les fibres sont des fibres de verre.

6. - Orthèse de jambe selon la revendication 2 ou 3, **caractérisée en ce que** les fibres sont des fibres de polyester.

7. - Orthèse de jambe selon l'une des revendications 2 à 6, **caractérisée en ce que** les fibres sont constituées par une mélange de différents types de fibres.

8. - Orthèse de jambe selon l'une des revendications 1 à 7, **caractérisée en ce que** le matériau du ressort d'appui (1) est un matériau plat.
